# EUROPEAN PATENT SPECIFICATION

(11) **EP 3 842 522 B1**
(45) Date of publication and mention of the grant of the patent: **15.10.2025**
(21) Application number: 19851533.0
(22) Date of filing: 21.08.2019
(51) Int. Cl.: A61K 40/11, A61K 40/22, A61K 40/41, C12N 5/0783

(54) **METHOD FOR PRODUCING REGULATORY T CELLS**
VERFAHREN ZUR HERSTELLUNG VON REGULATORISCHEN T-ZELLEN
PROCÉDÉ DE PRODUCTION DE LYMPHOCYTES T RÉGULATEURS

(30) Priority: 22.08.2018 JP 2018155507
(43) Date of publication of application: 30.06.2021
(73) Proprietor: The University of Osaka, Suita-shi, Osaka 565-0871 (JP)
(72) Inventor: SAKAGUCHI, Shimon, Suita-shi, Osaka 565-0871 (JP); MIKAMI, Norihisa, Suita-shi, Osaka 565-0871 (JP)
(74) Representative: Hoffmann Eitle
(86) International application number: PCT/JP2019/032637
(87) International publication number: WO 2020/040198

(56) References cited:
- WO-A1-2012/096376
- WO-A1-2018/037105
- JP-A- 2009 195 228
- JP-A- 2010 531 138
- JP-A- 2011 519 271
- JP-A- 2013 501 057
- US-A1- 2006 286 067
- US-A1- 2009 136 470
- US-A1- 2012 076 805
- NIKOLOULI EIRINI ET AL: "Alloantigen-Induced Regulatory T Cells Generated in Presence of Vitamin C Display Enhanced Stability of Foxp3 Expression and Promote Skin Allograft Acceptance", FRONTIERS IN IMMUNOLOGY, vol. 8, 28 June 2017 (2017-06-28), XP055830913, ISSN: 1664-3224, DOI: 10.3389/fimmu.2017.00748
- EI WAKAMATSU ET AL: "Strong TCR stimulation promotes the stabilization of Foxp3 expression in regulatory T cells induced in vitro through increasing the demethylation of Foxp3 CNS2", BIOCHEMICAL AND BIOPHYSICAL RESEARCH COMMUNICATIONS, vol. 503, no. 4, 18 September 2018 (2018-09-18), Amsterdam NL, pages 2597 - 2602, XP055687319, ISSN: 0006-291X, DOI: 10.1016/j.bbrc.2018.07.021
- VARUN SASIDHARAN NAIR ET AL: "Vitamin C Facilitates Demethylation of the Foxp3 Enhancer in a Tet-Dependent Manner", THE JOURNAL OF IMMUNOLOGY, vol. 196, no. 5, 1 March 2016 (2016-03-01), US, pages 2119 - 2131, XP055687324, ISSN: 0022-1767, DOI: 10.4049/jimmunol.1502352
- WAKAMATSU E. ET AL.: "Strong TCR stimulation promotes the stabilization of Foxp3 expression in regulatory T cells induced in vitro through increasing the demethylation of Foxp3 CNS2", BIOCHEMICAL AND BIOPHYSICAL RESEARCH COMMUNICATIONS, vol. 503, no. 4, 18 September 2018 (2018-09-18), pages 2597 - 2602, XP055687319, ISSN: 0006-291X, DOI: 10.1016/j.bbrc.2018.07.021
- SASIDHARAN NAIR V. ET AL.: "Vitamin C facilitates demethylation of the Foxp3 enhancer in a Tet-dependent manner", THE JOURNAL OF IMMUNOLOGY, vol. 196, no. 5, 1 March 2016 (2016-03-01), pages 2119 - 2131, XP055687324, ISSN: 0022-1767, DOI: 10.4049/jimmunol.1502352

## Description

### TECHNICAL FIELD

This application provides a method for inducing regulatory T cells from peripheral T cells.

### BACKGROUND

A key feature of CD25-positive CD4-positive regulatory T cells inherent in the immune system is to specifically express a transcriptional factor Foxp3. Deletion or mutation of Foxp3 may impair the generation, differentiation, and immunosuppressive function of regulatory T cells. Foxp3 gene has been regarded as a master gene for the generation and function of regulatory T cells based on the experimental results showing that normal T cells exerted immunosuppressive function when Foxp3 is ectopically expressed therein. Mutations in Foxp3 gene have been reported to inhibit the generation of regulatory T cells in most cases, resulting in abnormal regulation of immune responses to self and non-self antigens.

It has been reported that Foxp3-expressing induced regulatory T cells that have the functions and phenotypes similar to those of naturally occurring regulatory T cells are experimentally generated by culturing mouse CD4-positive T cells in a medium comprising IL2 and TGFβ in the presence of anti-CD3 antibody and anti-CD28 antibody and then culturing the cells in a medium comprising IL2 and TGFβ (Patent literature 1). In addition, several methods have been proposed to induce regulatory T cells from peripheral T cells (Patent literatures 2-5). The expression of Foxp3 in the induced regulatory T cells is, however, unstable and hence it is difficult to employ the induced regulatory T cells in clinical applications.

Recent studies have shown that the instability of Foxp3 expression in the induced regulatory T cells is primarily due to the differences in epigenetic regulation of Foxp3 gene. Epigenetics is defined as the changes in gene expression or phenotype that are inherited after the cell division without any changes in the DNA sequence. Epigenetic regulation of gene expression refers to the regulation of gene expression due to the modification of DNA or chromatin without any changes in the DNA sequence.

It has become clear that lineage-specific regulation of gene expression depends on regulation by transcription factors and epigenetic regulation. Epigenetic regulation in a regulatory T cell includes demethylation regions specific to regulatory T cells in the regions regulating the expression of genes encoding various functional proteins specific to regulatory T cells such as Foxp3. It is believed that the expression of genes specific to regulatory T cells such as Foxp3 gene is unstable in conventional induced regulatory T cells because the demethylation regions specific to regulatory T cells in the genes specific to regulatory T cells such as Foxp3 gene remain methylated in conventional induced regulatory T cells (Non-patent literatures 1 and 2). In addition, existing methods of inducing demethylation, e.g. by using ascorbic acid, cannot induce regulatory T cell-specific demethylations in human T cells (Non-patent literature 3).

US 2012/0076805 A1 describes to the use of PD-Ls (e.g., PD- L1 and/or PD-L2) to induce or differentiate naive T cells, and in particular CD4⁺ Foxp3⁻ T cells, toward a regulatory T cell phenotype.

US 2006/0286067 A1 describes methods for generating T regs using a two-stage culturing of PBMCs, instead of using a continuous culture with TGF-B and a cytokine.

US 2009/0136470 A1 describes to contact blood cells or T cells with an amount of TGF-beta or TGF-beta analogue and a retinoic acid receptor agonist, or an amount of a retinoid X receptor (RXR) or peroxisome proliferator activated receptor-gamma (PPAR- gamma) agonist, sufficient to stimulate or increase differentiation to regulatory T cells.

### Citation List

### Patent Literature

[Patent Literature 1] JP2009-195228A
[Patent Literature 2] JP2010-4853A
[Patent Literature 3] JP2011-519271A
[Patent Literature 4] JP2010-531138A
[Patent Literature 5] WO2012/096376

### Non-Patent Literature

[Non-Patent Literature 1] Floess et al. PLos Biol. (2007) 5(2) :169-178
[Non-Patent Literature 2] Ohkura et al. Immunity (2012) 785-799
[Non-Patent Literature 3] Kasahara et al. Int. Immunol. (2017) 29(10):457-469

### SUMMARY OF INVENTION

### TECHINACAL PROBLEM

An object of this application is to provide a method for inducing regulatory T cells from peripheral T cells in *vitro.* The regulatory T cells induced by the methods of this application function as regulatory T cells and are stable. The regulatory T cells can be used as regulatory T cells for the treatment and prevention of various immune diseases, organ transplant rejection, and inflammatory diseases such as autoimmune diseases, and for the induction of transplant immune tolerance.

### SOLUTION TO PROBLEM

The inventors found for the first time that functionally stable regulatory T cells can be induced from peripheral T cells by the step of culturing the peripheral T cells in the presence of CD3 stimulation without CD28 stimulation.

The present invention is defined in the appended claims.

In a first aspect, this application provides a method for producing regulatory T cells from mammalian-derived peripheral T cells, comprising the step of culturing the mammalian-derived peripheral T cells in a medium comprising TGFβ and IL-2 in the presence of CD3 stimulation and in the absence of CD28 stimulation wherein the medium further comprises ascorbic acid. In this aspect, the medium may further comprise retinoic acid.

In this aspect, the method may comprise the step of further culturing the obtained cell culture in a medium comprising IL-2.

In a second aspect, this application provides a method comprising the steps of culturing mammalian-derived peripheral T cells in a medium comprising IL-2 in the presence of CD3 stimulation and CD28 stimulation, and culturing the obtained culture in a medium comprising IL-2, prior to subjecting the mammalian-derived peripheral T cells to the method of the first aspect. Accordingly, the second aspect of this application provides a method for producing regulatory T cells, comprising the steps of (1-1) culturing mammalian-derived peripheral T cells in a medium comprising IL-2 in the presence of CD3 stimulation and CD28 stimulation, (1-2) culturing the obtained culture in a medium comprising IL-2, and (2) culturing the obtained culture in a medium comprising TGFβ and IL-2 in the presence of CD3 stimulation and in the absence of CD28 stimulation.

In the second aspect, step (2) is the same step as in the first aspect, and the medium may further comprise ascorbic acid. The method may further comprise the step of culturing the obtained culture in a medium comprising IL-2. The second aspect is particularly suitable for use in inducing regulatory T cells from human peripheral T cells.

### EFFECTS OF THE INVENTION

Regulatory T cells can be induced from peripheral T cells *in vitro* by the methods of this application. The regulatory T cells obtained by the methods of this application are functionally stable. In other words, functionally stable regulatory T cells that stably express Foxp3, which is a master gene of regulatory T cells, can be induced from peripheral T cells of mammals including humans by the methods of this application.

### BRIEF DESCRIPTION OF DRAWINGS

[Fig. 1] Results of bisulfite sequencing for regulatory T cell-specific genes in regulatory T cells induced from mouse naïve T cells in the presence or absence of anti-CD28 antibody.
[Fig. 2] Percentage and number of Foxp3-positive cells when regulatory T cells were induced from mouse naïve T cells in the presence or absence of anti-CD28 antibody.
[Fig. 3] Superiority of DNA demethylation when combined with ascorbic acid in the same culture system as in Example 1.
[Fig. 4] Foxp3 stability in regulatory T cells generated in the same culture system as in Example 2 and then restimulated with anti-CD3/anti-CD28 antibodies.
[Fig. 5] *In vitro* suppressive activity of regulatory T cells generated in the same culture system as in Example 2.
[Fig. 6] The expressions of surface molecular markers on regulatory T cells generated in the same culture system as in Example 2 and then restimulated with anti-CD3/anti-CD28 antibodies.
[Fig. 7] Foxp3 stability in regulatory T cells generated by the same method as in Example 1 and further cultured for stabilization in a medium only comprising IL-2.
[Fig. 8] RNA-seq data of regulatory T cells generated by the same method as in Example 1 and regulatory T cells generated by the same method as in Example 1 and further cultured for stabilization for 4 days. The expression of the genes above the dotted line was significantly upregulated in the regulatory T cells cultured for stabilization. The expression of the genes below the dotted line was significantly downregulated in the regulatory T cells cultured for stabilization.
[Fig. 9] Regulatory T cells cultured for stabilization were administered to wild-type mice, and Foxp3 expression in the transferred regulatory T cells was analyzed 2 weeks after the administration. In the right figure, the concentrations of anti-CD28 antibody/ascorbic acid are shown in parentheses.
[Fig. 10] Results of analyzing the activation states of T cells in DO11.10/RagKO mice transferred with regulatory T cells generated from DO11.10-positive T cells and cultured for stabilization, and then immunized with OVA/Alum. The concentrations of anti-CD28 antibody/ascorbic acid are shown in parentheses.
[Fig. 11] Wild mice were transferred with regulatory T cells that were generated from effector T cells collected from DNFB-immunized mice and cultured for stabilization as described in Example 1. The phenotype of the recipient mice was then analyzed when inducing skin inflammation by using DNFB. The concentrations of anti-CD28 antibody/ascorbic acid are shown in parentheses.

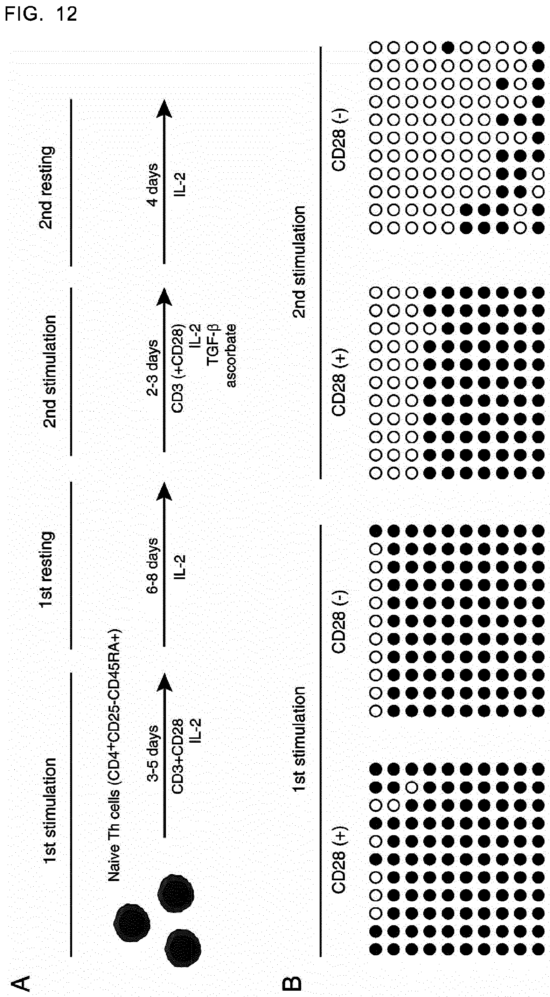

[Fig. 12] Results of analyzing DNA demethylation by bisulfite sequencing in regulatory T cells induced from human T cells. Human T cells were stimulated in a medium comprising anti-CD3/anti-CD28 antibodies and IL-2 for 3 days, and then cultured for pretreatment in a medium only comprising IL-2 for 4 days. The pretreated cells or untreated human T cells were stimulated in a medium comprising anti-CD3 antibody, IL-2, TGFβ, anti-cytokine antibody, and anti-FASL antibody in the presence or absence of anti-CD28 antibody for 3 days, and then Foxp3-positive cells were collected and subjected to bisulfite sequencing.
[Fig. 13] Superiority of DNA demethylation when combined with ascorbic acid.

### DETAILED DESCRIPTION

In this specification and claims, when a numerical value is accompanied with the term "about", the value is intended to include the range of ± 10% of that value. For example, "about 20" includes "18 to 22". A numerical range includes all values between the two endpoints and the values of both endpoints. When a numerical range is accompanied with "about", "about" is applied to the two endpoints. Accordingly, for example, "about 20 to 30" includes "18 to 33".

In this application, regulatory T cells are produced from peripheral T cells. In this specification and claims, "peripheral T cells" refers to T cells outside the thymus and can be obtained from peripheral blood, lymph nodes, spleen, etc. In this application, "peripheral T cells" may be included in a cell population and are not necessarily be isolated. Cell fractions comprising T cells and various lymphocytes, such as peripheral blood mononuclear cells (PBMC), may be used.

Peripheral T cells include naive regulatory T cells, CD4-positive T cells, and CD8-positive T cells. Regulatory T cells may be induced from a culture comprising multiple types of T cells or induced from specific cells, such as CD4-positive T cells and CD8-positive T cells, isolated from the cells. Regulatory T cells may also be induced from isolated T cells specific to a given antigen.

In this specification and claims, the expression "CD4 positive" or "CD4⁺" refers to CD4-positive CDB-negative single positive cells, unless otherwise indicated. The expression "CD8 positive" or "CD8⁺" refers to CD4-negative CD8-positive single positive cells.

In this specification and claims, "regulatory T cells" refers to cells that are positive for at least CD4, CD25, and Foxp3.

In this specification and claims, "mammals" are humans and non-human animals such as mice, rats, cattle, horses, pigs, sheep, monkeys, dogs, cats, and birds, and are not particularly limited. For example, "mammals" are mice or humans.

In this specification and claims, the terms "CD28 stimulation" and "CD3 stimulation" refer to substances that specifically stimulate CD28 receptors and CD3 receptors on cells, respectively. For example, CD28 stimulation and CD3 stimulation include anti-CD28 agonist antibody and anti-CD3 agonist antibody, respectively. In this specification and claims, "anti-CD28 antibody" and "anti-CD3 antibody" refer to anti-CD28 agonist antibody and anti-CD3 agonist antibody, respectively. Anti-CD28 agonist antibody and anti-CD3 agonist antibody may be prepared by using commercially available products for research reagents or produced by conventional methods.

Anti-CD3 antibody and anti-CD28 antibody can be derived from animals such as mice, rabbits, goats, and cattle, or humans.

In the methods of this application, antibodies can be added to a medium or immobilized on the inner wall of a culture vessel or on the surface of an insoluble carrier. The insoluble carrier may be a material that can physically or chemically bind to anti-CD3 antibody or anti-CD28 antibody, and may be insoluble in aqueous solution. The materials that can physically adsorb anti-CD3 antibody and anti-CD28 antibody include synthetic resins such as polystyrene, polyethylene terephthalate, polycarbonate, and polypropylene, and glass. The shape of the insoluble carrier is not particularly limited and may be, for example, a plate, a bead, or a container.

In the methods of this application, a basal medium for animal cell culture supplemented with necessary factors is used for cell culture. Examples of basal media for animal cell culture which can be used in this application include Iscove's modified Eagle's Medium, Ham's F12, MEM Zinc Option, IMEM Zinc Option, IMDM, Medium 199, Eagle's Minimum Essential Medium (EMEM), αMEM, Dulbecco's modified Eagle's Medium (DMEM), RPMI 1640, Fischer's medium, and a mixture thereof.

The basal medium may contain serum (e.g. fetal bovine serum (FBS)) or may be a serum-free medium. If necessary, the serum-free medium may comprise one or more serum substitutes such as albumin, bovine serum albumin (BSA), transferrin, apo-transferrin, KnockOut Serum Replacement (KSR) (Invitrogen), which is a serum substitute used for culturing ES cells, N2 supplement (Invitrogen), B27 supplement (Invitrogen), a fatty acid, insulin, collagen precursor, a trace element, 2-mercaptoethanol, 3'-thiolglycerol, and monothioglycerol. In addition, the basal medium may comprise one or more substances such as lipid (e.g. chemically defined lipid concentrate), an amino acid, L-glutamine, GlutaMAX (Invitrogen), a nonessential amino acid (NEAA), a vitamin (e.g. nicotinamide and ascorbic acid), a growth factor, an antibiotic (e.g. penicillin and streptomycin), an anti-oxidant, pyruvic acid, a buffering agent, an inorganic salt, and equivalents thereof.

In one embodiment, RPMI 1640 medium comprising serum and 2-mercaptoethanol is exemplified as the basic medium.

In the methods of this application, cells may be cultured under the condition generally used for animal cell culture. The culture temperature is, but not limited to, about 30 to 40°C, preferably about 37°C. The culture is preferably performed under CO₂-containing air atmosphere. The CO₂ concentration is preferably about 2 to 5%.

In a first aspect, provided is a method for producing regulatory T cells from mammalian-derived peripheral T cells, comprising the step of culturing the mammalian-derived peripheral T cells in a medium comprising TGFβ and IL-2 in the presence of CD3 stimulation and in the absence of CD28 stimulation , wherein the medium further comprises ascorbic acid.

When anti-CD3 antibody is used as the CD3 stimulation in the methods of this application, the anti-CD3 antibody may be added directly to the medium or immobilized on the inner wall of the culture vessel or on the surface of an insoluble carrier. The amount of anti-CD3 antibody varies depending on the titer and origin of the antibody, but it may be appropriately determined to provide the stimulation sufficient to induce regulatory T cells.

Examples of TGFβ used in the first aspect include TGFβ1, TGFβ2, and TGFβ3. For example, TGFβ is TGFβ1. The concentration of the TGFβ may be appropriately determined by those skilled in the art and is not particularly limited. When TGFβ1 is used as the TGFβ, its concentration in the medium may be 0.25 to 25 ng/mL, e.g. about 2.5 ng/mL.

The concentration of IL-2 in the medium used in the first aspect is, but not limited to, about 5 U/mL to about 500 U/mL, e.g. about 50 U/mL.

Retinoic acid may be further added to the medium used in this aspect. The concentration of ascorbic acid is, but not limited to, about 1 to about 100 µg/mL, e.g. about 10 µg/mL.

The culture period may be appropriately determined by those skilled in the art and is not particularly limited. For example, the culture period may be about 3 days.

Regulatory T cells having regulatory T cell-specific demethylation states can be induced by culturing mammalian-derived peripheral T cells in a medium comprising TGFβ and IL-2 in the presence of CD3 stimulation and in the absence of CD28 stimulation. The regulatory T cell-specific demethylation states in the obtained regulatory T cells can be confirmed, for example, by the demethylations in the CNS2 site of Foxp3.

In the first aspect, the method preferably comprises the step of further culturing the cells in a medium comprising IL-2 after culturing the mammalian-derived peripheral T cells in the medium comprising TGFβ and IL-2 in the presence of CD3 stimulation and in the absence of CD28 stimulation. In the "step of further culturing the cells in a medium comprising IL-2", any stimulations to T cell receptors such as CD3 stimulation and CD28 stimulation are not added, while stabilizing factors for regulatory T cells such as mTOR inhibitors, e.g. rapamycin, may be added.

The concentration of IL-2 is, but not limited to, for example about 100 to 500 U/ml. The culture period in the step of further culturing the cells in the medium comprising IL-2 may be determined by those skilled in the art and is not particularly limited. For example, the culture period may be about 4 to 10 days.

The induced regulatory T cells having regulatory T cell-specific demethylation states can be proliferated by culturing the cells in the presence of IL-2 without any stimulation.

In a second aspect of this application, the method comprises the step of culturing mammalian-derived peripheral T cells in a medium comprising IL-2 in the presence of CD3 stimulation and CD28 stimulation, and further culturing the stimulated culture in a medium comprising IL-2, before the step of culturing the mammalian-derived peripheral T cells in a medium comprising TGFβ and IL-2 in the presence of CD3 stimulation and in the absence of CD28 stimulation. Accordingly, the second aspect of this application provides a method for producing regulatory T cells, comprising the steps of (1-1) culturing mammalian-derived peripheral T cells in a medium comprising IL-2 in the presence of CD3 stimulation and CD28 stimulation, (1-2) culturing the obtained culture in a medium comprising IL-2, and (2) culturing the obtained culture in a medium comprising TGFβ and IL-2 in the presence of CD3 stimulation and in the absence of CD28 stimulation.

In this aspect, step (1-1) is the step of culturing mammalian-derived peripheral T cells in a medium comprising IL-2 in the presence of CD3 stimulation and CD28 stimulation.

The concentration of IL-2 in the medium in step (1-1) may be, but not limited to, for example about 100 to 500 U/mL. In this aspect, when anti-CD3 antibody is used as the CD3 stimulation and anti-CD28 antibody is used as the CD28 stimulation, each of the anti-CD3 antibody and the anti-CD3 antibody may be added directly to the medium or immobilized on the inner wall of the culture vessel or on the surface of an bead. The amount of each of anti-CD3 antibody and anti-CD28 antibody varies depending on the titer and origin of each antibody and may be appropriately determined. The culture period in step (1-1) may be appropriately determined by those skilled in the art and is not particularly limited. For example, the culture period may be about 3 to 5 days.

Step (1-2) is the step of further culturing the peripheral T cells cultured in the medium comprising IL-2 in the presence of CD3 stimulation and CD28 stimulation, in a medium comprising IL-2.

the "medium comprising IL-2" in step (1-2) does not comprise T-cell receptor stimulation such as CD3 stimulation and CD28 stimulation. The concentration of IL-2 in the medium is, but not limited to, for example about 100 to 500 U/ml. The culture period in this step may be appropriately determined by those skilled in the art and is not particularly limited. For example, the culture period may be about 5 to 10 days.

In step (2), the cell culture obtained in step (1-2) is cultured in a medium comprising TGFβ and IL-2 in the presence of CD3 stimulation and in the absence of CD28 stimulation. This is the same step as in the first aspect of this application. The medium may further comprise ascorbic acid. The cells can be further cultured in a medium comprising IL-2 to obtain a stable regulatory T cell culture containing induced regulatory T cells.

The second aspect is particularly suitable for use in inducing regulatory T cells from human peripheral T cells. Human peripheral T cells are, for example, human peripheral blood mononuclear cells. When regulatory T cells are induced from human peripheral T cells, one or more of anti-cytokine neutralizing antibodies such as anti-IFNγ antibody, anti-IL-4 antibody, anti-IL-6 antibody, and anti-FasL neutralizing antibody, and CTLA4-Ig, may be added to the medium in step (2). The amount of each component may be appropriately determined, and for example about 1 µg/mL.

Regulatory T cells may be isolated from the obtained cell culture containing regulatory T cells by conventional methods based on cell surface markers specific to regulatory T cells, e.g. by collecting Foxp3-positive fractions by using a cell sorter. If desired, regulatory T cells with specific antigenic properties may be isolated.

Regulatory T cells having regulatory T cell-specific demethylation states similar to those of naturally occurring regulatory T cells can be induced from mammalian peripheral T cells by the methods described in the first and second aspects of this application.

Regulatory T cells obtained by the methods of this invention are expected to be used for treating inflammatory diseases in humans, such as autoimmune diseases and allergies. The regulatory T cells are also expected to be used for inducing immune tolerance in transplantation.

### EXAMPLE

This invention is described in more detail referring to following examples. This invention, however, is not limited by those Examples in any way.

In the following examples, RPMI 1640 (containing 10% FCS (v/v), 60 µg/mL penicillin G, 100 µg/mL streptomycin, and 0.1 mM 2-mercaptoethanol) was used as a basal medium.

### Bisulphite Sequencing

Genomic DNA was obtained from induced cells, treated with MethylEasy Xceed Rapid DNA Bisulphite Modification Kit (Human Genetic Signatures), and then examined for demethylations in genes specific to regulatory T cells. The demethylations in each gene were analyzed by using known methods and primers (Floess et al. (2007) PloS Biology Volume 5, Issue 2, e38, and Ohkura et al. (2012) Immunity 37(5) 785-799).

For the analysis in mouse Foxp3 CNS2 region, 5'-ATTTGAATTGGATATGGTTTGT-3' (SEQ ID NO: 1) was used as the primer on the forward side, and 5'-AACCTTAAACCCCTCTAACATC-3' (SEQ ID NO: 2) was used as the primer on the reverse side . For the analysis in human Foxp3 CNS2 region, 5'-TTGGGTTAAGTTTGTTGTAGGATAG-3' (SEQ ID NO: 3) was used as the primer on the forward side, and 5'-ATCTAAACCCTATTATCACAACCCC-3' (SEQ ID NO: 4) was used as the primer on the reverse side.

### Collection of the fractions of each T cell

Foxp3-eGFP (eFox) reporter mice (Ito et al. (2014) Science 346, 363-368) were used. The lymph node cells were obtained from Foxp3-eGFP (eFox) reporter mice and CD4⁺ GFP⁺ cells were sorted by FACSAriaII (BD) to obtain the fractions of naturally occurring regulatory T cells (nTreg). CD4⁺ GFP⁻CD44^{low} CD62L^{high} cells were sorted to obtain the fractions of naive T cells. In addition, the fractions of CD4⁺ GFP⁻ CD44^{high} CD62L^{low} cells were obtained and used as effector/memory T cells.

Human CD4⁺ T cells were isolated from PBMCs of healthy individuals. The enriched PBMCs were labeled with anti-CD4, anti-CD25, and anti-CD45RA monoclonal antibodies for flow cytometry on ice for 30 minutes. The fractions of CD4⁺ CD25⁻CD45RA⁺ cells were obtained from the labeled cells by FACSAriaII.

The antibodies used in the examples are as follows.

**Table 1**

| Antibodies | Clone | Application | Vendor |
|---|---|---|---|
| anti-mouse CD16/32 | 93 | FCM | BD |
| anti-mouse CD25 | PC61 | FCM | BD |
| anti-mouse CD28 | 37.51 | Stimulation | BD |
| anti-mouse CD3ε | 145-2C11 | Stimulation | BD/Biolegend/ produced in-house |
| anti-mouse CD4 | RM4-5 | FCM | BD/eBioscience |
| anti-mouse CD62L | MEL-14 | FCM | BD |
| anti-DO11.10 | KJ1-26 | FCM | BD |
| anti-human CD25 | M-A251 | FCM | BD |
| anti-human CD4 | RPA-T4 | FCM | BD |
| anti-human CD45RA | HI100 | FCM | BD |
| anti-human FOXP3 | 236A/E7 | FCM | eBioscience |
| anti-human/mouse CD44 | IM7 | FCM | BD |
| anti-human CD3 | UCHT1 | stimulation | BD |
| anti-human CD28 | CD28.2 | stimulation | BD |

### Example 1

### Production of regulatory T cells from mouse T cells

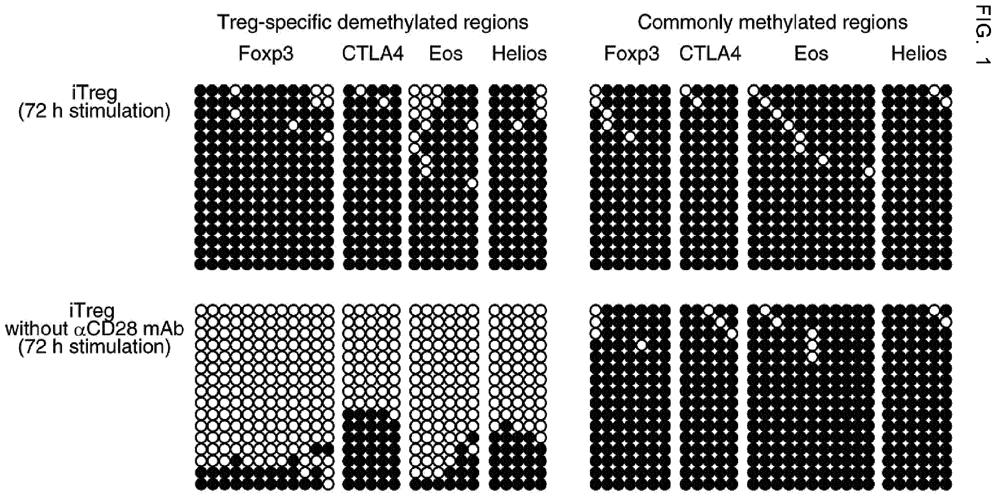

Mouse naive T cells were obtained from lymph nodes of eFox reporter mice by FACS. The mouse naive T cells were seeded at 2 x 10⁵ cells/well in 96-well plates (Nunc) and stimulated in the basal medium comprising anti-CD3 antibody (immobilized in the container by adding 100 µL of the antibody per well at 10 µg/ml and incubating it for 60 minutes at room temperature), hIL-2 (50 U/ml), and hTGFβ1 (2.5 ng/ml) in the presence or absence of anti-CD28 antibody (added to the medium at the final concentration of 1 µg/ml) for 3 days. Foxp3-positive cells were then isolated by FACS and subjected to bisulfite sequencing (Fig. 1). We analyzed regulatory T cell-specific demethylation sites and commonly methylation sites in Foxp3, CTLA4, Eos, and Helios. When the medium contained anti-CD28 antibody, demethylations did not occur in most regulatory T cell-specific demethylation sites. In contrast, demethylations occurred when the medium did not contain anti-CD28 antibody, confirming that the obtained regulatory T cells had the properties similar to those of naturally occurring regulatory T cells.

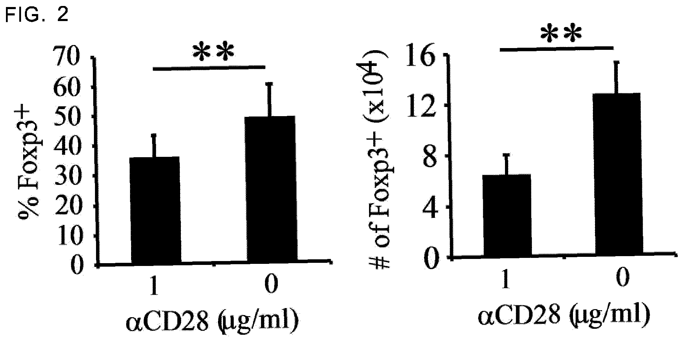

The Foxp3 expressions in the cells cultured in the above experimental system were compared by FACS. It was confirmed that the ratio and number of Foxp3-expressing cells were increased in the cells obtained under the condition without anti-CD28 antibody (Fig. 2).

### Example 2

### Superiority of ascorbic acid for demethylation

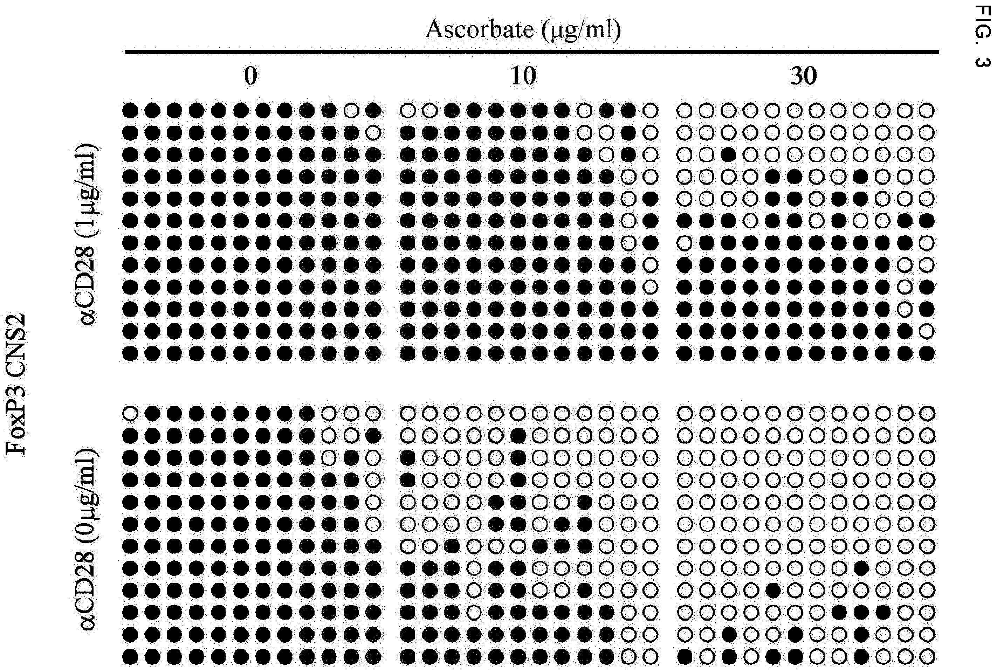

In the same experimental system as in Example 1, the effect of ascorbic acid was examined by adding 10 µg/ml or 30 µg/ml of ascorbic acid to the basal medium. The results are shown in Fig. 3. It was confirmed that the degree of the demethylations was further increased by adding ascorbic acid to the medium.

### Example 3

### Stability, surface molecular marker expression, and in vitro suppressive activity of regulatory T cells

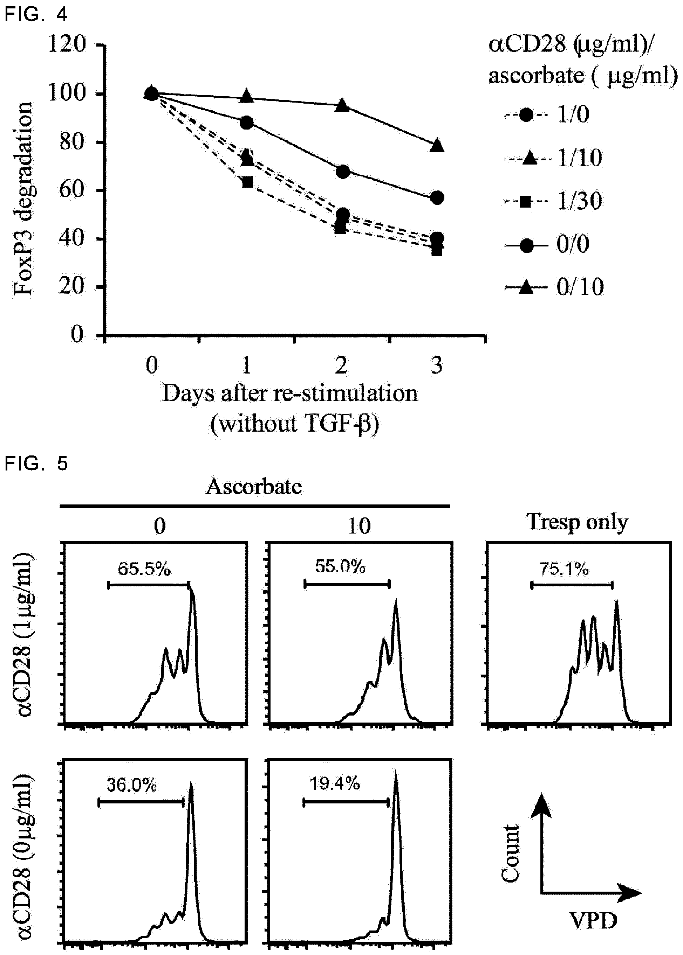

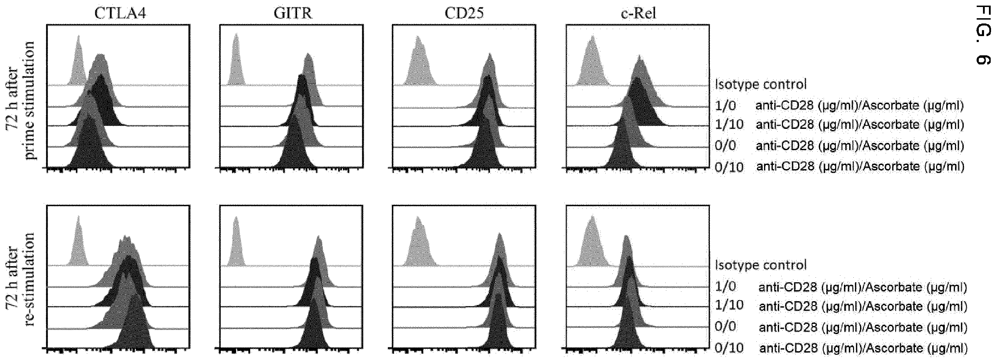

Regulatory T cells were obtained by the same experimental system as in Example 2. CD3/CD28 Dynabeads were added to the obtained regulatory T cells at a ratio of the number of the cells : the beads = 1 : 1 to restimulate the cells with anti-CD3/anti-CD28 antibodies. The stability of Foxp3 was examined at the time of the restimulation and 1, 2, and 3 days after the restimulation (Fig. 4). It was confirmed that the regulatory T cells induced without CD28 stimulation showed little change in the expression level of Foxp3 after the restimulation and stably expressed Foxp3. We also examined the expressions of surface molecular markers at the time of the restimulation and 3 days after the restimulation (Fig. 6). CTLA4, GITR, CD25, and c-Rel, which are surface molecular markers of regulatory T cells, were all stably expressed even 3 days after the restimulation.

Next, we examined the *in vitro* suppressive activity of regulatory T cells generated in the same experimental system as in Example 2 (Fig. 5). CFSE-labeled responder T cells (5 x 10⁴), the generated regulatory T cells (5 x 10⁴), and antigen-presenting cells (2 x 10⁴) were mixed and cultured in the presence of anti-CD3 antibody (1 µg/ml) for 3 days (Fig. 5). CFSE intensity was then analyzed by FACS. The antigen-presenting cells were obtained by sorting MHC Class II(+) cells from mouse lymph nodes by FACS. When responder T cells mount an immune response, CFSE intensity shows multiple weak peaks due to the proliferation of the cells, while when the immune response is suppressed, the CFSE intensity remains a single strong peak. It was confirmed that the regulatory T cells induced without CD28 stimulation strongly suppressed the immune response of the responder T cells. Accordingly, it was confirmed that the regulatory T cells induced without CD28 stimulation were functionally stable as compared with regulatory T cells obtained by conventional methods.

### Example 4

### Culture of regulatory T cells for stabilization

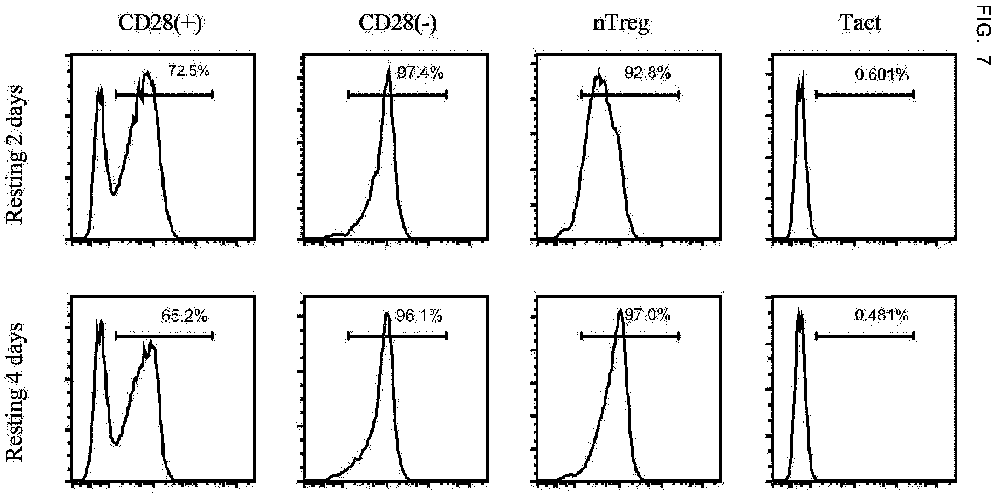

Regulatory T cells generated by the method described in Example 1 were further cultured in the basal medium only comprising IL-2 for 2 or 4 days (culture for stabilization). It was confirmed that regulatory T cells induced without CD28 stimulation stably expressed Foxp3 after the culture for stabilization (Fig. 7).

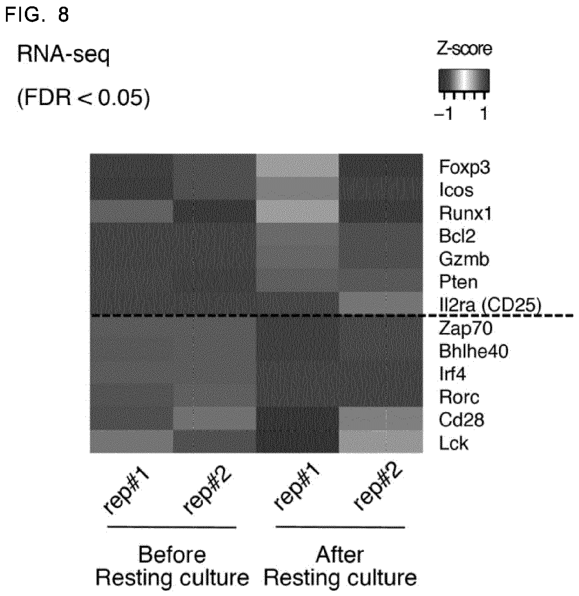

We compared RNA-seq data of regulatory T cells generated by the method described in Example 1 with those of regulatory T cells generated by the method described in Example 1 and then cultured for stabilization for another 4 days (Fig. 8). RNA-seq was performed by using known methods (Kitagawa et al. (2017) Nat. Immunol. 18, 173-183). Row Tag Count data mapped to UCSC Ref genome data (mm9) were tested by negative binomial distribution modeling (DESeq2; Love, 2014) to calculate False Discovery Rate (FDR). The expressions of Foxp3, CD25, and Granzyme B (Gzmb), a major inhibitory molecule of Treg, were significantly upregulated in the regulatory T cells cultured for stabilization (FDR < 0.05; the genes above the dotted line in Fig. 8). The upregulated expression of Bcl2 may suggest the increase in *in vivo* viability. The genes that were significantly downregulated in the regulatory T cells cultured for stabilization included many TCR signal-related molecules, such as Lck and Zap70, and their downstream effector T cell differentiation factors, e.g. Th17-related factors RorgT (Rorc) and Bhlhe40 (the genes below the dotted line in Fig. 8). The expressions of genes related to T follicular helper cells (Tfh) such as Bcl6 were also downregulated (data not shown).

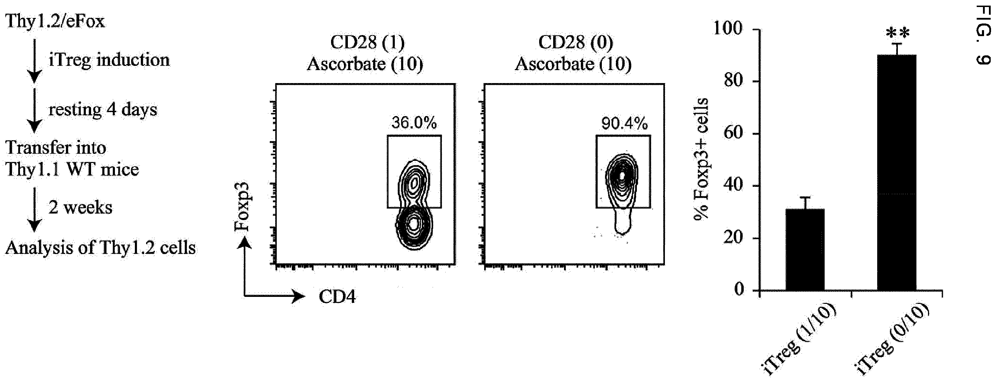

Regulatory T cells were generated from Thy1.2/eFox reporter mice by the method described in Example 1 and cultured for stabilization for 4 days as described above. Foxp3-positive cells (2 x 10⁵ cells) were isolated from the cells cultured for stabilization by FACS and administered to Thy1.1/wild-type mice via the tail veins. Two weeks later, the transferred Thy1.2 cells were collected from the lymph nodes and analyzed for Foxp3 expression (Fig. 9). It was confirmed that the regulatory T cells induced without CD28 stimulation and then cultured for stabilization stably expressed Foxp3 even 2 weeks after the administration to in *vivo.*

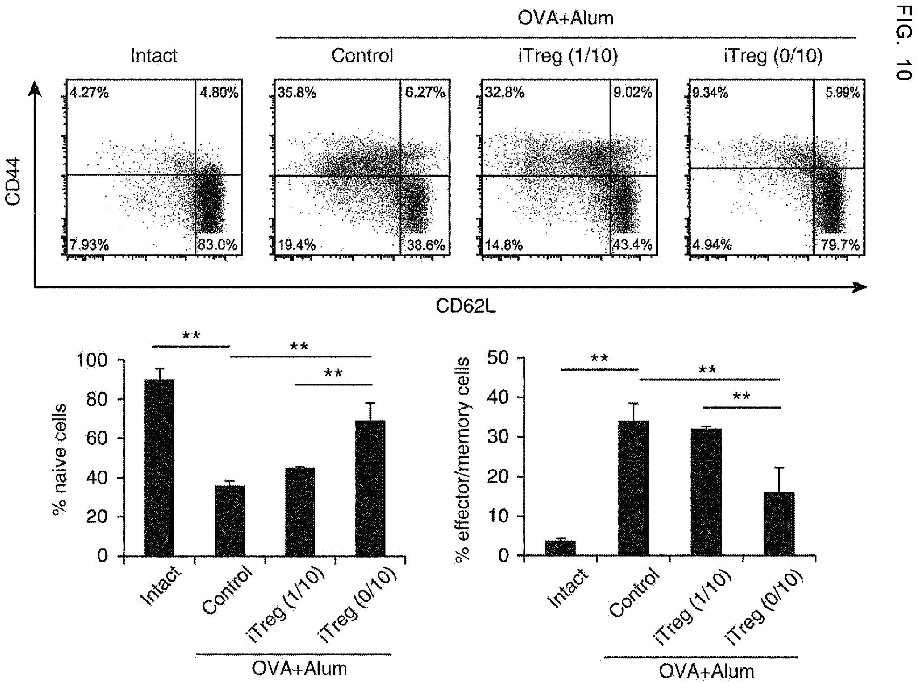

CD4⁺ cells collected from DO11.10 mice were converted to regulatory T cells by the method described in Example 1 and then cultured for stabilization for 4 days. DO11.10 mouse is a transgenic mice that highly expresses OVA-specific T cells. Accordingly, regulatory T cell fractions containing OVA-specific regulatory T cells can be generated by the above method. These regulatory T cells (2 x 10⁵) were administered to DO11.10/RagKO mice via the tail veins, and the activation states of T cells were analyzed upon the immunization with OVA/Alum (Fig. 10). It was confirmed that T cells in the DO11.10/RagKO mice were activated upon the OVA stimulation, and the immune reactions of the T cells were strongly suppressed by the regulatory T cells induced without CD28 stimulation and cultured for stabilization, compared to the regulatory T cells induced with CD28 stimulation.

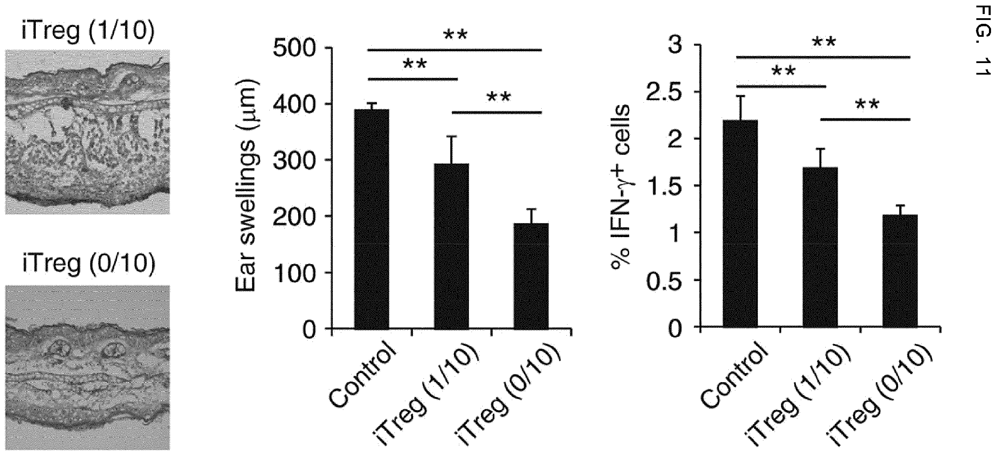

2,4-Dinitrofluorobenzene (DNFB) was applied to the abdomens of wild-type mice. Five days later, effector cells (CD4⁺ GFP- CD44^{high} CD62L^{low} cells) were collected. The collected effector cells were converted into regulatory T cells by the method described in Example 1 and cultured for stabilization for 4 days. The regulatory T cells (2 x 10⁵) were administered to wild-type mice via the tail veins, and DNFB was applied to the abdomens of wild-type mice for sensitization. Six days later, DNFB was applied to the auricles to induce skin inflammation. Two days later, the phenotype of the tissue was examined and FACS analysis was performed for T cells collected from the regional lymph nodes (Fig. 11). It was confirmed that the regulatory T cells induced without CD28 stimulation and then cultured for stabilization suppressed the skin inflammation, compared to the regulatory T cells induced with CD28 stimulation.

### Example 5

### Production of regulatory T cells from human T cells

Human CD4⁺ CD25⁻ CD45RA⁺ T cells (human T cells) were stimulated in the basal medium comprising anti-CD3 antibody, anti-CD28 antibody, and IL-2 for 3 days. The cells were then cultured for pretreatment in the basal medium supplemented with only IL-2 for 4 days. The pretreated cells or untreated human T cells were stimulated in the basal medium comprising IL-2 (50 U/ml), TGF-β (2.5 ng/ml), anti-IFN-g antibody (the final concentration of 1 µg/mL), anti-IL-4 antibody (the final concentration of 1 µg/mL), anti-IL-6 antibody (the final concentration of 1 µg/mL), anti-Fas ligand antibody (the final concentration of 1 µg/mL), and CTLA4-Ig (the final concentration of 1 µg/mL) in the presence of anti-CD3 antibody (immobilized in the container by adding 100 µL of the antibody per well at 10 µg/ml and incubating it for 60 minutes at room temperature) and in the presence or absence of anti-CD28 antibody (added to the medium at the final concentration of 1 µg/ml) for 3 days. Foxp3-positive cells were then collected by FACS and subjected to bisulfite sequencing in order to examine the demethylations in CNS2 region of Foxp3. The results are shown in Fig. 12.

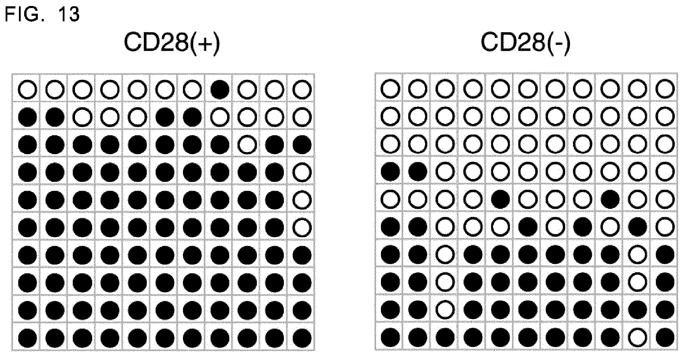

Few demethylations in Foxp3 occurred under the condition without the culture for pretreatment. On the other hand, the demethylations in Foxp3 CNS2 region were observed in the cells cultured without CD28 stimulation after the culture for pretreatment. Accordingly, it was confirmed that stable regulatory T cells can be produced by pre-culturing human T cells for pretreatment. In addition, the demethylation effect of ascorbic acid was also observed as in mice (Fig. 13).

### Industrial applicability

Regulatory T cells can be stably induced from peripheral T cells by the methods of this application. Regulatory T cells induced by the methods of this application are expected to be used for treating and preventing various immune diseases, organ transplant rejection, and inflammatory diseases such as autoimmune diseases, and for inducing transplant immune tolerance.

## Claims

1. A method for producing regulatory T cells, comprising the step of culturing mammalian-derived peripheral T cells in a medium comprising TGFß and IL-2 in the presence of CD3 stimulation and in the absence of CD28 stimulation, wherein the medium
further comprises ascorbic acid.

2. The method according to claim 1, further
comprising the step of culturing the cell culture obtained in claim 1 in a medium comprising IL-2.

3. A method for producing regulatory T cells, comprising the steps of:
(1-1) culturing mammalian-derived peripheral T cells in a medium comprising IL-2 in the presence of CD3 stimulation and CD28 stimulation;
(1-2) culturing the obtained culture in a medium comprising IL-2; and
(2) culturing the obtained culture in a medium
comprising TGFß and IL-2 in the presence of CD3 stimulation and in the absence of CD28 stimulation.

4. The method according to claim 3, wherein the medium in step (2) further comprises ascorbic acid.

5. The method according to claim 3 or 4, further comprising the step of culturing the culture obtained in step (2) in a medium comprising IL-2.

6. The method according to any one of claims 3 to 5, wherein the mammalian-derived peripheral T cells are human-derived peripheral T cells.

7. The method according to claim 6, wherein the human-derived peripheral T cells are human peripheral blood mononuclear cells.

## Patentansprüche

1. Verfahren zur Herstellung von regulatorischen T-Zellen, umfassend den Schritt der Kultivierung von peripheren T-Zellen, die von Säugetieren abstammen, in einem Medium, das TGFβ und IL-2 umfasst, in Gegenwart von CD3-Stimulation und in Abwesenheit von CD28-Stimulation, wobei das Medium weiter Ascorbinsäure umfasst.

2. Verfahren nach Anspruch 1, weiter umfassend den Schritt der Kultivierung der in Anspruch 1 erhaltenen Zellkultur in einem Medium, das IL-2 umfasst.

3. Verfahren zur Herstellung von regulatorischen T-Zellen, umfassend die Schritte:
(1-1) Kultivierung von peripheren T-Zellen, die von Säugetieren abstammen, in einem Medium, das IL-2 umfasst, in Gegenwart von CD3-Stimulation und CD28-Stimulation;
(1-2) Kultivierung der erhaltenen Kultur in einem Medium, das IL-2 umfasst; und
(2) Kultivierung der erhaltenen Kultur in einem Medium, das TGFβ und IL-2 umfasst, in Gegenwart von CD3-Stimulation und in Abwesenheit von CD28-Stimulation.

4. Verfahren nach Anspruch 3, wobei das Medium in Schritt (2) weiter Ascorbinsäure umfasst.

5. Verfahren nach Anspruch 3 oder 4, weiter umfassend den Schritt der Kultivierung der in Schritt (2) erhaltenen Kultur in einem Medium, das IL-2 umfasst.

6. Verfahren nach irgendeinem der Ansprüche 3 bis 5, wobei die von Säugetieren abstammenden peripheren T-Zellen periphere T-Zellen sind, die von Menschen abstammen.

7. Verfahren nach Anspruch 6, wobei die von Menschen abstammenden peripheren T-Zellen mononukleäre Zellen des menschlichen peripheren Blutes sind.

## Revendications

1. Un procédé de production de cellules T régulatrices, comprenant l'étape de culture de cellules T périphériques dérivées de mammifères dans un milieu comprenant du TGFß et de l'IL-2 en présence d'une stimulation CD3 et en l'absence de stimulation CD28, dans lequel le milieu comprend en outre de l'acide ascorbique.

2. Le procédé selon la revendication 1, comprenant en outre l'étape de culture de la culture cellulaire obtenue dans la revendication 1 dans un milieu comprenant de l'IL-2.

3. Le procédé de production de cellules T régulatrices, comprenant les étapes de :
(1-1) culture des cellules T périphériques dérivées de mammifères dans un milieu comprenant de l'IL-2 en présence d'une stimulation CD3 et d'une stimulation CD28 ;
(1-2) culture de la culture obtenue dans un milieu comprenant de l'IL-2 : et
(2) culture de la culture obtenue dans un milieu comprenant du TGFß et de l'IL-2 en présence d'une stimulation CD3 et en l'absence d'une stimulation CD28.

4. Le procédé selon la revendication 3, dans lequel le milieu de l'étape (2) comprend en outre de l'acide ascorbique.

5. Le procédé selon la revendication 3 ou 4, comprenant en outre l'étape de culture de la culture obtenue à l'étape (2) dans un milieu comprenant de l'IL-2.

6. Le procédé selon l'une quelconque des revendications 3 à 5, dans lequel les cellules T périphériques dérivées de mammifères sont des cellules T périphériques d'origine humaine.

7. Le procédé selon la revendication 6, dans lequel les cellules T périphériques d'origine humaine sont des cellules mononucléaires de sang périphérique humain.
